## Europäisches Patentamt

## European Patent Office

## Office européen des brevets

(19)

(11) Publication number : **0 225 909**
**B1**

(12) # EUROPEAN PATENT SPECIFICATION

(45) Date of publication of patent specification :
02.11.89

(51) Int. Cl.⁴ : **A 61 F   5/453**

(21) Application number : 86903648.3

(22) Date of filing : 14.05.86

(86) International application number :
**PCT/SE 86/00226**

(87) International publication number :
**WO/8606620 (20.11.86 Gazette 86/25)**

(54) A method for the manufacture of an incontinence protector.

(30) Priority : 14.05.85 SE 8502402

(43) Date of publication of application :
24.06.87 Bulletin 87/26

(45) Publication of the grant of the patent :
02.11.89 Bulletin 89/44

(84) Designated contracting states :
AT BE CH DE FR GB IT LI NL SE

(56) References cited :
FR--A--   556 020
FR--A-- 2 312 228
SE--A--85 004 927
SE--B--   426 206
SE--B--   440 180

(73) Proprietor : Mölnlycke AB
S-405 03 Göteborg (SE)

(72) Inventor : NIKLASSON, Berndt, Michael
Lingonvägen 17 B
S-435 00 Mölnlycke (SE)

(74) Representative : Alfredson, Stig et al
H. ALBIHNS PATENTBYRA AB Box 3137
S-103 62 Stockholm (SE)

Jouve, 18, rue St-Denis, 75001 Paris, France

## Description

The present invention relates to a method of manufacturing incontinence protectors for men, in which is incorporated an absorbent layer and a liquid-impermeable outer layer.

For slightly incontinent men, i. e. those involuntarily leaking small amounts of urine only dropwise, there have so far existed no efficient incontinence protectors.

Slight incontinence is a concealed handicap affecting many people. A large group of slightly incontinent men encompasses those having prostatic insufficiency. Even after having undergone prostatic surgery, a problem still remaining for this group of men is usually drip incontinence, which has up to now caused a great deal of mental suffering due to the lack of appropriate protective appliances.

Conventional diapers or other types of incontinence protectors for heavily incontinent individuals are naturally unacceptable for men discharging urine only dropwise, in which case there would be no need to carry around large and clumsy protectors.

However, incontinent protectors intended for slightly incontinent men are known per se. GB-A-2 059 779 teaches such a protector formed of two side walls affixed to their edges while leaving an opening at one side edge, said protector having one pointed and one obtuse corner. When applying the protector with the opening over penis, the obtuse corner situated opposite the opening is pressed against the body, the edges of the opening thereby being folded out towards the sides. The portion located beneath the obtuse corner and in the region around the pointed corner forms a conically shaped receptacle accommodating the user's penis during use of the protector.

The protector, which is surrounded by a plastic layer completely encasing it, has an inner layer of absorbent material and a friction increasing coating applied to the surface of the plastic layer. It is formed externally of an easily flexible plastic film folded inwardly with edge flaps at the edges of the opening.

According to said publication, the user wearing the protector described will have his organ completely enclosed by a liquid- and vapour-impermeable receptacle, which makes a device of this kind much too hot and tight, and thus unpleasant to wear.

A further disadvantage is that the construction of this protector makes it too stiff to feel comfortable.

The Swedich Patent Specification 416 264 discloses a receptacle for slightly incontinent men. The receptacle consists of a tubular, fine-meshed textile hosiery which is impregnated with a liquid-repellent agent. The absorbent material is applied in small parcels to be laid inside the receptacle.

This protector, which is intended to be threaded onto the male organ like a stocking, is completely useless for men having retarded penis. Since male individuals suffering from drip incontinence are mostly elderly men, among which a retarded penis is especially common, the protector described in the last-mentioned specification will not serve its purpose.

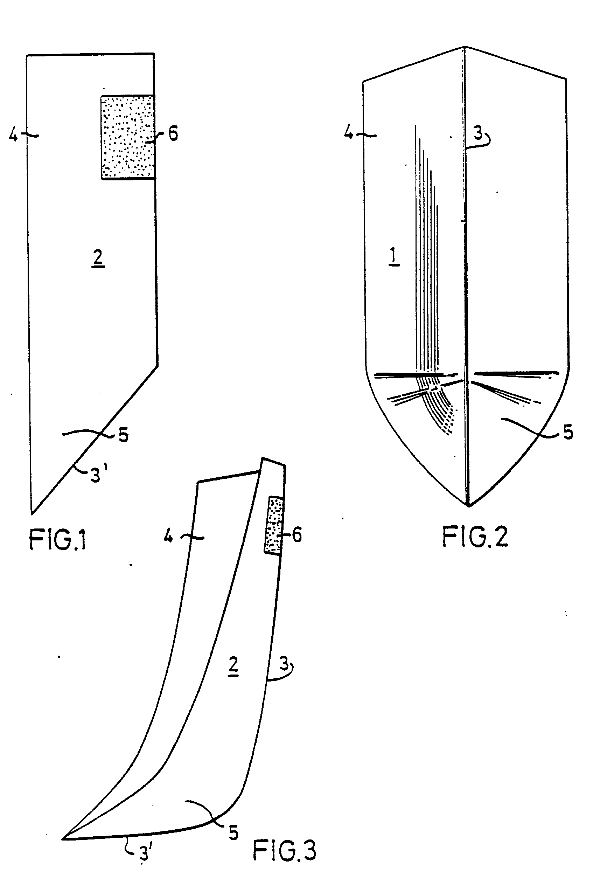

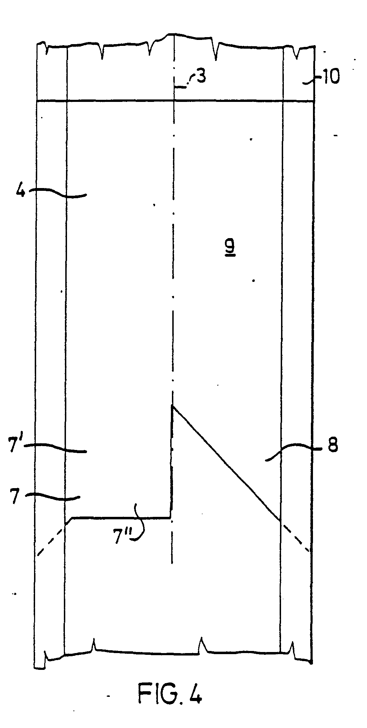

Furthermore, the type of protector disclosed in said Swedish Patent Specification 416 264 is unsuitable even for men with normal penis due to the obvious difficulty in finding a fastening means keeping the protector fixed in position during use. The waistband shown in Fig 4 of said patent specification will naturally cause discomfort and does not seem to fulfill its function in a satisfactory manner. According to the embodiment shown in Fig 1, the protector should be affixed in the region around the penis root with the aid of adhesive tape, which would certainly make removal of the protector painful for the user.

With the present invention there is accomplished a protector intended for slightly incontinent men which with regard to use is quite superior to previously known appliances.

The inventive method of manufacturing such protectors is primarily distinguished in that blank material for the protectors is advanced in the form of a continuous web preferably consisting of a laminate composed of an absorbent layer and a liquid-impermeable layer, or of several individual layers, and is provided with two laterally separated flaps extending in the longitudinal direction of the protectors at one end portion of each single web portion intended as a protector, the inner edges of the flaps joining each other at a point of the longitudinal centerline of the material web; that the web of material is folded together along said centerline; and that the flaps being brought together upon said folding are joined along a line extending from said point obliquely towards the free flap ends and forming an acute angle to the centerline while constituting the bottom edge of the finished protector, the connected flaps of which, when unfolding the protector, presenting a downwardly tapering, cup-like configuration.

In this manner there is obtained an airy and comfortable protector, which does not encase the user's organ but which instead forms a ventilated splash guard. Beyond being well-adapted to the bodily shape of the wearer, the design of the protector with a downwardly tapering, cup-like portion also provides a satisfactory shield against downward splash.

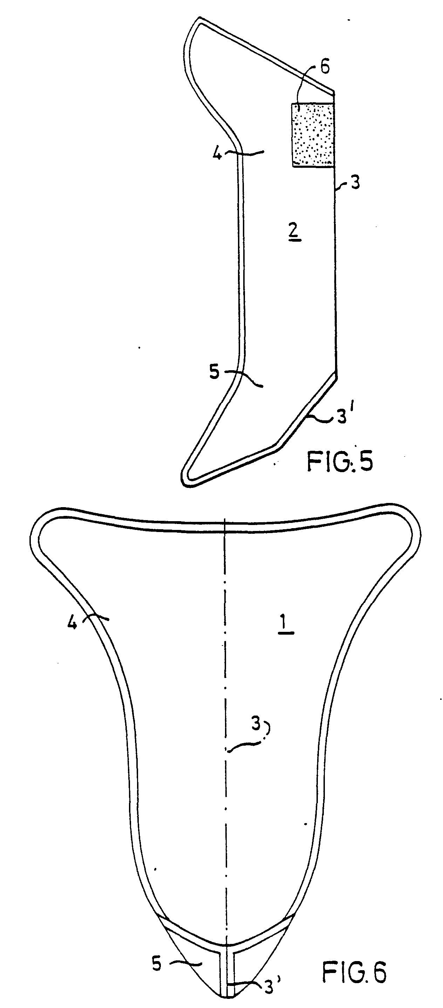

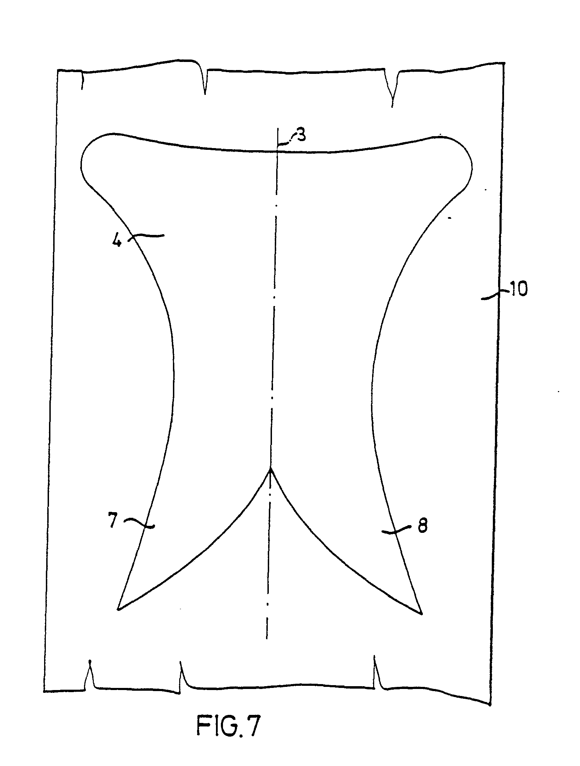

The invention will be described in more detail below with reference to a few exemplary embodiments shown in the accompanying drawings, of which Fig 1 is a side view of a first embodiment of an incontinence protector in a folded-together condition and manufactured in accordance with the inventive method; Fig 2 shows the protector of Fig 1 in its spread-out condition for use as seen in a direction straight towards the interior of the protector; Fig 3 is a lateral perspective view of the protector shown in Fig 2 ; and Fig 4 illustrates the method of manufacturing the protectors shown in Figs 1-3 ; whereas Fig 5 is a side view showing a second embodiment of the inventive protector when folded together ; Fig 6 showing the protector of Fig 5 in its spread-out state of use as viewed straight towards the interior of the protector ; Fig 7 finally schematically illustrating the method of manufacturing the protectors shown in Figs 5 and 6.

The incontinence protector illustrated in Figs 1-3 consists of a liquid-permeable layer 1 of fiber fabric, a liquid-impermeable layer 2 of plastics, and an absorbent layer which is surrounded by the two outer layers 1 and 2.

The protector is elongated and symmetric with regard to a longitudinal centerline 3. The protector shown in Fig 1 is folded together around the centerline 3 and has an upper portion 4 and a lower portion 5. A piece of material 6 of two-side adhesive tape is affixed to the plastic layer at the upper portion 4 of the protector.

The folded layer shown in Fig 1 extends continuously along the lines 3 and 3'.

When unfolding said protector to its state of use as shown in Figs 2 and 3, there is formed an upper, shield-like portion 4 for application to the body of the user so as to cover his penis and scrotum, and a lower, cup-like portion 5 intended to bend a distance in under penis and scrotum.

Due to its design with a downwardly tapering cup-shaped portion 5, the incontinence protector is given a soft, bodily adapted and rounded-off configuration at its bottom portion. The cup-shaped, tapering portion 5 will not give rise to chafing in the crotch region, which is, however, often the case with conventional diapers.

As is most clearly seen in Fig 3 the cupshaped portion 5 does not form any receptacle intended to enclose penis and scrotum during use. Instead there is created an airy splash guard for dropwise incontinent individuals. In addition to making the protector well adapted to the body of the wearer, its cup-forming design provides a splash guard working in all directions : upwards and sidewards by the shield portion 4, and downwards by the cup-shaped portion 5.

Fig 4 shows schematically the inventive method of manufacturing the protector according to Figs 1-3. The basic product consists of planar, oblong pieces of absorption material designed according to the Figure with a shield-like upper portion 4 and two flaps 7, 8 at one end portion. One of said flaps 8 has triangular shape, while the other one has a flap portion 7' congruent with the flap 8. As shown in the Figure, this portion 7' and the flap 8 are symmetrically positioned in relation to the longitudinal centerline 3 of the blank, which is indicated in Fig 4 by a dash-dotted line. The flap 7 further has an extension 7" protruding from the portion 7'.

Absorption layers 9 according to Fig 4 are formed to be fed as a continuous web (not shown) over a plastic film 10 intended to constitute the liquid-impermeable outer layer. A web of fiber fabric (not shown) is applied on top of the absorption material layers 9. The width of the plastic film web 10 is greater than that of the absorption material layers 9, and the protruding portions are folded inwards over the edge portion of the absorption material layers to be affixed thereto.

In the subsequent manufacturing process, the web composed of different layers of material is folded along the centerline 3, while the flap portion 7" is simultaneously pushed in under the flap 8. Individual incontinence protectors are formed by the web being cut off between each single protector, and by the plastic film being sheared and welded for connection with the oblique boundary edges facing each other upon folding of the flap 8 and the flap portion 7'. By means of this welded seal, designated by 3' in Fig 1, there is obtained a cup-shaped bottom portion 5 when unfolding the folded-together protector.

The protector shown in Figs 5 and 6 agrees in all essentials with the embodiment according to Figs 1-3, and the details corresponding to similar parts in Figs 1-3 have therefore been given the same reference numerals. A comparison between Figs 1-3 and Figs 5-6 will reveal a few variations as to the geometrical configuration. As is evident from Fig 6, there is formed also in this embodiment an upper, shield-like portion 4 and a lower, cup-shaped portion 5.

Fig 7 illustrates schematically the inventive method of manufacturing the incontinence protector according to Figs 5 and 6. Absorption material layers 9 with an upper portion 4 and having flaps 7 and 8 at one end portion are shaped. The material pieces 9 are symmetrical with regard to the longitudinal centerline 3. A continuous web of such absorption material pieces is fed between a liquid-impermeable web 10 of material layer, preferably a plastic film, and a liquid-permeable web of material layer (not shown), preferably of fiber fabric. The two outer layers are cut off around the absorption material piece and are simultaneously sealed so as to form a casing surrounding it.

When folding the material layers along the centerline 3, the flaps 7 and 8 are brought to face one another. Subsequent to folding along the edge portion designated by 3' in Figs 5 and 6, these flaps are joined together for producing in this manner a cup-shaped bottom portion 5 when unfolding the folded protector to its state of use.

The invention is not restricted to the exemplary embodiments described above, since a plurality of modifications are conceivable within the scope of the following patent claims.

The material used for the protector may vary. The outer layer 2 should be liquid-impermeable, but could of course very well be vapour-permeable. The plastic film can be replaced by a hydrophobic, vapour-permeable layer of fiber fabric.

With regard to comfort and discrete use, the absorption layer should be soft and thin. Highly absorbent material could be included to advantage. Alternatively, the absorption layer could be composed of one or more layers of tissue.

In the embodiments described above, the cup-shaped portion is formed by two flaps, separated in their flat condition, which are bent in towards one another and joined. Another method of providing a cup-shaped portion on a planar blank is the application of elastic pre-stress means thereto, said means in their released condition making said portion cupshaped.

The cup shape obtained on the portion formed by the two curved flaps can be enhanced with the aid of elastic members applied under pretension in connection with the flaps being connected.

The incontinence protectors manufactured in accordance with the inventive method has been described above as particularly useful for slightly incontinent men. The design of the protector, distinguished by the bodily adapted cup shape at its underside, however, renders it useful also for heavily incontinent users because of the possibility of making it comparatively thick without feeling awkward, for example by using cellulose fiber for the absorption body.

## Claims

1. A method of manufacturing incontinence protectors for men in which is incorporated an absorbent layer and a liquid-impermeable outer layer (2), characterized in that blank material for the protectors is advanced in the form of a continuous web preferably consisting of a laminate composed of an absorbent layer (9) and a liquid-impermeable layer, or of several individual layers, and is provided with two laterally separated flaps (7, 8) extending in the longitudinal direction of the protectors at one end portion of each single web portion intended as a protector, the inner edges of the flaps joining each other at a point of the longitudinal centerline (3) of the material web; that the web of material is folded together along said centerline; and that the flaps (7, 8) being brought together upon said folding are joined along a line extending from said point obliquely towards the free flap ends and forming an acute angle to the centerline (3) while constituting the bottom edge of the finished protector, the connected flaps (7, 8) of which, when unfolding the protector, presenting a downwardly tapering, cup-like configuration.

2. A method according to Claim 1, characterized in that individual, elongate pieces of material intended to serve as absorption layers for the protectors, are provided at one of their end portions with two flaps (7, 8) extending in the longitudinal direction of the layer and having their inner boundary lines starting from a common point on the longitudinal centerline (3) of the material piece while having in the direction towards the opposite ends of the flaps a mutual, preferably continually increasing distance; that said pieces of material are applied between a liquid-impermeable web of plastics or hydrophobic vapour-permeable material, and a liquid-permeable layer preferably of fiber fabric; that the web thus consisting of three layers is folded together, the liquid-permeable layer thereby facing inwards; that the two outer layers are cut off between each single piece of absorption material to be joined together at the edges of the absorption layer for creating a casing surrounding this layer, the flaps (7, 8) abutting each other then being joined together for producing the bottom edge of the completed protector.

3. A method according to Claim 2, characterized in that the pieces of absorption material are produced by airlaying cellulose full pulp, possibly in combination with highly absorbent material.

## Patentansprüche

1. Verfahren zur Herstellung von Inkontinenz-schutzeinlagen für Männer, welche Inkontinenz-schutzeinlagen eine absorbierende Schicht und eine flüssigkeitsundurchlässige Außenschicht (2) einverleibt enthalten, dadurch gekennzeichnet, daß das Rohlingsmaterial für die Schutzeinlagen in der Form einer kontinuierlichen Bahn vorwärtsbewegt wird, welche Bahn vorzugsweise aus einem Laminat besteht, welches aus einer absorbierenden Schicht (9) und einer flüssigkeitsundurchlässigen Schicht, oder aus mehreren Einzelschichten zusammengesetzt ist, und daß dieses Rohlingsmaterial mit zwei seitlich voneinander getrennten Ansätzen (7, 8) ausgestattet ist, welche sich in der Längsrichtung der Schutzeinlagen an einem Endteil von jedem einzelnen Abschnitt der Bahn erstrecken, der für eine Schutzeinlage vorgesehen ist, wobei die Innenränder der Ansätze an einem Punkt der in der Längsrichtung verlaufenden Mittellinie (3) der Bahn aus Material zusammenkommen; daß die Bahn aus Material längs dieser Mittellinie zusammengefaltet wird; und daß die bei diesem Falten zusammengebrachten Ansätze (7, 8) längs einer Linie miteinander verbunden werden die sich von diesem Punkt schräg in Richtung auf die freien Enden der Ansätze erstreckt und mit der Mittellinie (3) einen spitzen Winkel bildet, während sie den unteren Rand der fertigen Schutzeinlage bildet, deren miteinander verbundene Ansätze (7, 8), wenn die Schutzeinlage auseinandergefaltet wird, eine sich nach unten verjüngende, becherartige Konfiguration darbieten.

2. Verfahren nach Anspruch 1, dadurch gekennzeichnet, daß einzelne, langgestreckte Stücke aus Material, die dazu bestimmt sind, als Absorptionsschichten für die Schutzeinlagen zu dienen, an einem ihrer Endteile mit zwei Ansätzen (7, 8) ausgestattet werden, die sich in der Längsrichtung der Schicht erstrecken, und deren innere Begrenzungslinien von einem gemeinsamen Punkt auf der in der Längsrichtung verlaufenden Mittellinie (3) des Stückes aus Material ausgehen, während sie in der Richtung auf die einander gegenüberliegenden Enden der Ansätze einen gegenseitigen, vorzugsweise kontinuierlich zunehmenden Abstand voneinander aufweisen ; daß diese Stücke aus Material zwischen einer flüssigkeitsundurchlässigen Bahn aus Kunststoff oder einem hydrophoben, dampfdurchlässigen Material und einer flüssigkeitsdurchlässigen Schicht, vorzugsweise aus einem Faserstoff, eingelegt werden ; daß die somit aus drei Schichten bestehende Bahn zusammengefaltet wird, derart, daß die flüssigkeitsdurchlässige Schicht nach innen gekehrt ist ; daß die zwei Außenschichten jeweils zwischen den einzelnen Stücken aus Absorptionsmaterial abgeschnitten werden, um miteinander an den Rändern der Absorptionsschicht unter Schaffung einer diese Schicht umgebenden Umhüllung verbunden zu werden, wobei dann die mit ihren Enden zusammengefügten Ansätze (7, 8) miteinander verbunden werden, um den unteren Rand der fertigen Schutzeinlage zu erzeugen.

3. Verfahren nach Anspruch 2, dadurch gekennzeichnet, daß die Stücke aus Absorptionsmaterial durch Ablegen von Zellulosevollzellstoff, gegebenenfalls in Kombination mit einem hochgradig absorbierenden Material, in einem Luftstrom erzeugt werden.

**Revendications**

1. Procédé pour fabriquer des protections contre l'incontinence masculine, dans lesquelles sont incorporées une couche absorbante et une couche extérieure (2) imperméable aux liquides, caractérisé par le fait que : une matière brute destinée aux protections est avancée sous la forme d'une bande continue consistant, de préférence, en un stratifié composé d'une couche absorbante (9) et d'une couche imperméable aux liquides, ou de plusieurs couches individuelles, deux ailes (7, 8) séparées latéralement et s'étendant dans la direction longitudinale des protections étant formées à une des parties d'extrémité de chaque tronçon individuel de bande destiné à former une protection, les bords intérieurs des ailes se rejoignant en un point de l'axe longitudinal (3) de la bande de matière ; que la bande de matière est pliée sur elle-même le long de l'axe longitudinal ; et que les ailes (7, 8), amenées l'une contre l'autre lors du pliage, sont assemblées le long d'une ligne s'étendant depuis ledit point, obliquement en direction des extrémités libres des ailes et formant un angle aigu avec l'axe (3) tout en constituant le bord inférieur de la protection achevée, dont les ailes reliées (7, 8), quand on déplie la protection, présentant une configuration en forme de cuvette et s'effilant vers le bas.

2. Procédé selon la revendication 1, caractérisé par le fait que : les pièces individuelles allongées de matière, destinées à servir de couches absorbantes pour les protections, sont pourvues à une de leurs parties d'extrémité de deux ailes (7, 8) s'étendant dans la direction longitudinale de la couche et ayant leurs lignes de délimitation intérieure commençant depuis un point commun se trouvant sur l'axe longitudinal (3) de la pièce de matière tout en présentant, en direction des extrémités opposées des ailes, une distance mutuelle augmentant de préférence de façon continue ; que lesdites pièces de matière sont appliquées entre une bande de matière plastique imperméable aux liquides ou d'une matière hydrophobe et perméable aux vapeurs, et une couche perméable aux liquides, de préférence un tissu fibreux ; que la bande consistant ainsi en trois couches est pliée sur elle-même, la couche perméable aux liquides se trouvant ainsi vers l'intérieur ; que les deux couches extérieures sont sectionnées entre chaque paire de pièce de matière absorbante pour être assemblées l'une à l'autre aux bords de la couche absorbante pour créer une enveloppe entourant cette couche, les ailes (7, 8) buttant l'une contre l'autre étant ensuite assemblées l'une à l'autre pour former le bord inférieur de la protection achevée.

3. Procédé selon la revendication 2, caractérisé par le fait que les pièces de matière absorbante sont réalisées à partir d'une pâte aérée formée entièrement de cellulose, éventuellement en combinaison avec une matière extrêmement absorbante.

4 ～

6

2

5

3'

FIG.1

4 ～                    3 ～

1

5

FIG.2

4 ～                6

2      3 ～

5

3'      FIG.3

FIG. 4

FIG.5

FIG.6

FIG.7